# EUROPEAN PATENT APPLICATION

(11) **EP 4 852 053 A1**
(43) Date of publication of application: **09.12.2026**
(21) Application number: 25180073.6
(22) Date of filing: 02.06.2025
(51) Int. Cl.: H01J 49/00, G16B 40/10

(54) **ANALYTICAL INSTRUMENTS AND DATA PROCESSING METHODS**

(71) Applicant: HighChem s.r.o, 821 09 Bratislava (SK)
(72) Inventor: Zsemlye, Gábor, 93101 Samorín (SK); Lutisan, Juraj, 01861 Belusa (SK)
(74) Representative: Pecharová, Petra

(57) **Abstract**

A control unit for processing data acquired by an analytical instrument is provided, the control unit configured to: receive at least one mass spectrum of a sample, wherein the mass spectrum is acquired by an analytical instrument and comprises at least one peak having a peak position, represented by a m/z ratio, and a peak intensity; convert the at least one mass spectrum into a graph; apply a trained graph neural network on the graph; and output the result of the application of the trained graph neural network; wherein the graph comprises at least one node, each node representing, for a peak, peak position and peak intensity; and in case the spectrum of a sample comprises at least two peaks, at least one first edge connecting nodes representing neighbouring peaks in the spectrum.

## Description

### FIELD OF INVENTION

The disclosure relates to analytical instruments such as mass spectrometers and to processing data from such instruments.

### BACKGROUND

A typical mass spectrum shows intensity peaks for specific mass-to-charge, m/z, ratios. The identification of a compound or compounds present in the sample and their quantity is based on the peaks position, their intensity, and further information such as the peaks' relative positions or heights. The mass spectrum usually requires either an experienced user, or an automated tool, to identify the specific compound(s) present in the sample.

Once compound(s) of the sample are identified, it is possible to also identify the respective physical or chemical properties of the compound(s) present in the sample. However, the compound identification process may be lengthy, and rather than identifying only a single compound, it may return multiple results with varying degrees of uncertainty. The subsequent determination of physical or chemical properties of compound(s) present in the sample may also be affected. There is therefore a need for improvement.

### SUMMARY

In a first aspect, a control unit for processing data acquired by an analytical instrument is provided, the control unit configured to: receive at least one mass spectrum of a sample, wherein the mass spectrum is acquired by an analytical instrument and comprises at least one peak having a peak position, represented by a m/z ratio, and a peak intensity; convert the at least one mass spectrum into a graph; apply a trained graph neural network on the graph; and output the result of the application of the trained graph neural network; wherein the graph comprises at least one node, each node representing, for a peak, peak position and peak intensity; and in case the spectrum of a sample comprises at least two peaks, at least one first edge connecting nodes representing neighbouring peaks in the spectrum.

The following features may be (and in embodiments are) implemented, alone or in combination.

The result may comprise at least one of the following: at least one candidate component of the sample; at least one physical or chemical property of a component of the sample.

The control unit may be further configured to: assess whether, for each pair of peaks, the difference in peak positions corresponds to any one of a list of neutral mass losses, and in case a pair of nodes is assessed to represent peaks for which the difference in positions corresponds to a neutral mass loss, add a second edge connecting the pairs of nodes.

The control unit may be further configured to assign different weights or labels to the at least one first edge and the second edge or edges. All first edges may have the same weight or label. All second edges may have the same weight or label. Different second edges may have different weights or labels depending e.g. on which neutral mass loss they represent.

The control unit may be further configured to: receive a plurality of mass spectra of the sample, wherein each spectrum is acquired by the analytical instrument with a different value of a parameter; convert each of the plurality of spectra into a graph; assess the graphs to identify nodes represent peaks with corresponding peak positions, wherein the graph comprises at least one node, each node representing, for a peak, peak position and peak intensity, wherein the graph further comprises, in case the spectrum of a sample comprises at least two peaks, at least one first edge connecting nodes representing neighbouring peaks in the spectrum; the control unit being further configured to and add, where peaks with corresponding peak positions are identified, at least one third edge connecting nodes from different graphs, wherein the nodes connected by a third edge represent peaks with corresponding peak positions.

The parameter may be normalised collision energy, polarity or ion activation.

The control unit may be further configured to assign different weights or labels to the at least one first edge and the third edge or edges. All first edges may have the same weight or label. All third edges may have the same weight or label, e.g. depending on the value of the parameter, the relative intensity of the corresponding peaks (e.g. depending on the value of the parameter), and the like. The control unit may be further configured to assign different weights or labels to the second edge or edges and the third edge or edges.

The control unit may be further configured to receive at least a first mass spectrum and a second mass spectrum of the sample, wherein the first and second spectra respectively are acquired by the analytical instrument at a different MSn stage; convert the first and second spectra into a first and second graphs (comprising at least one node, each node representing, for a peak, peak position and peak intensity; and in case the respective spectrum comprises at least two peaks, at least one first edge connecting nodes representing neighbouring peaks in the spectrum); assess the first and second spectra and/or the first and second graphs to identify peaks, or nodes representing peaks, having corresponding peak positions in the first and second spectrum or the first and second graph, respectively; and add, where peaks with corresponding peak positions are identified, at least one fourth edge connecting nodes from the first and second graphs, wherein the connected nodes represent, in a respective MS(n-1) stage, a precursor for the respective MSn stage.

The control unit may be further configured to assign different weights or labels to the at least one first edge and the fourth edge or edges. All first edges may have the same weight or label. All fourth edges may have the same weight or label. The control unit may be further configured to assign different weights or labels to the first, second, third and/or fourth edge or edges, as appropriate.

The control unit may be further configured to control an analytical instrument to acquire mass spectra.

The features and embodiments of the control unit of the first aspect may be combined with each other as appropriate, even if such a combination is not explicitly stated above.

In a second aspect, a system comprising an analytical instrument and a control unit as specified above is provided.

In a third aspect, a method of processing data acquired by an analytical instrument is provided, the method comprising: receiving, by a control unit, at least one mass spectrum of a sample, wherein the mass spectrum is acquired by an analytical instrument and comprises peaks, each peak having a peak position, represented by a *m*/*z* ratio, and a peak intensity; converting, by the control unit, the at least one mass spectrum into a graph; applying, by the control unit, a trained graph neural network on the graph; and outputting, by the control unit, the result of the application of the trained graph neural network; wherein the graph comprises at least one node, each node representing, for a peak, peak position and peak intensity; and in case the spectrum of a sample comprises at least two peaks, at least one first edge connecting nodes representing neighbouring peaks in the spectrum.

The following features may be (and in embodiments are) implemented, alone or in combination. The method steps described below may be performed by the control unit of the first or third aspect.

The result may comprise at least one of the following: at least one candidate component of the sample; at least one physical or chemical property of a component of the sample.

The method may further comprise assessing whether, for each pair of peaks, the difference in peak positions corresponds to any one of a list of neutral mass losses, and in case a pair of nodes is assessed to represent peaks for which the difference in positions corresponds to a neutral mass loss, adding a second edge connecting the pairs of nodes representing peaks for which the difference in positions corresponds to a neutral mass loss.

The method may be further comprise assigning a different weight or label to the at least one first edge and the second edge or edges. All first edges may have the same weight or label. All second edges may have the same weight or label. Different second edges may have different weights or labels depending e.g. on which neutral mass loss they represent.

The method may further comprise: receiving a plurality of mass spectra of the sample, wherein each spectrum is acquired by the analytical instrument with a different value of a parameter; converting each of the plurality of spectra into a graph as specified above; assessing the graphs to identify nodes representing peaks with corresponding peak positions; and adding, where peaks with corresponding peak positions are identified, at least one third edge connecting nodes from different graphs, wherein the connected nodes represent peaks with corresponding peak positions.

The parameter may be normalised collision energy, polarity or ion activation.

The method may further comprise assigning different weights or labels to the at least one first edge and the third edge or edges. All first edges may have the same weight or label. All third edges may have the same weight or label. The method may further comprise assigning different weights or labels to the second edge or edges and the third edge or edges.

The method may further comprise: at least a first mass spectrum and a second mass spectrum of the sample, wherein the first and second spectra respectively are acquired by the analytical instrument at a different MSn stage; converting the first and second spectra into a first and second graphs (the graphs comprising at least one node, each node representing, for a peak, peak position and peak intensity; and in case the respective spectrum comprises at least two peaks, at least one first edge connecting nodes representing neighbouring peaks in the spectrum);
assessing the first and second spectra and/or the first and second graphs to identify peaks, or nodes representing peaks, having corresponding peak positions in the first and second spectrum or the first and second graph, respectively; and adding, where peaks with corresponding peak positions are identified, at least one fourth edge connecting nodes from the first and second graphs, wherein the connected nodes represent, in a respective MS(n-1) stage, a precursor for the respective MSn stage.

The method may further comprise assigning different weights or labels to the at least one first edge and the fourth edge or edges. All first edges may have the same weight or label. All fourth edges may have the same weight or label. The fourth edges may have different weights or labels, depending e.g. on which precursor they are associated with, which MSn stage they are associated with, etc. The method may be further configured to assign different weights or labels to the first and/or second and/or third and/or fourth edge or edges, as appropriate.

The method may further comprise controlling an analytical instrument to acquire mass spectra.

The different embodiments of the method of the third aspect may be combined with each other as appropriate, even if such a combination is not explicitly stated above.

In a fourth aspect, a method of training a graph neural network to process data acquired by an analytical instrument is provided, the method comprising: receiving, by a control unit, at least one mass spectrum, wherein the mass spectrum comprises peaks, each peak having a peak position, represented by a m/z ratio, and a peak intensity; receiving, by a control unit, other data for the at least one mass spectrum; converting, by the control unit, the at least one mass spectrum into a graph, the graph comprising at least one node, each node representing, for a peak, peak position and peak intensity, and in case the spectrum of a sample comprises at least two peaks, at least one first edge connecting nodes representing neighbouring peaks in the spectrum; the method further comprising applying, by the control unit, the graph neural network on the graph and the other data.

The following features may be (and in embodiments are) implemented, alone or in combination. The method steps described below may be performed by the control unit of the first or fourth aspect.

The other data may be at least one physical or chemical property of a compound represented by the mass spectrum.

The at least one mass spectrum may be a mass spectrum of a sample acquired by an analytical instrument, and the other data may be at least one library spectrum corresponding to a sample of the same composition.

The method may further comprise: converting, by the control unit, the at least one library spectra into a graph comprising: at least one node, each node representing, for a peak, peak position and peak intensity; and in case the spectrum of a sample comprises at least two peaks, at least one second edge connecting nodes representing neighbouring peaks in the spectrum; assessing, by the control unit, the graphs to identify nodes represent peaks with corresponding peak positions; and adding, by the control unit, at least one second edge connecting nodes from different graphs, wherein the nodes connected by the second edge represent peaks with corresponding peak positions.

The method may be further comprise assigning a different weight or label to the at least one first edge and the second edge or edges. All first edges may have the same weight or label. All third edges may have the same weight or label.

The method may further comprise assessing whether, for each pair of peaks in either the mass spectrum of a sample and a library spectrum or both, the difference in peak positions corresponds to any one of a list of neutral mass losses, and in case a pair of nodes in any one graph is assessed to represent peaks for which the difference in positions corresponds to a neutral mass loss, adding a third edge into the graph connecting the pairs of nodes representing peaks for which the difference in positions corresponds to a neutral mass loss.

The method may be further comprise assigning a different weight or label to the at least one first edge and the third edge or edges. The method may be further comprise assigning a different weight or label to the second edge or edges and the third edge or edges. All third edges may have the same weight or label. Different third edges may have different weights or labels depending e.g. on which neutral mass loss they represent.

The method may further comprise: receiving a plurality of mass spectra of the sample, wherein each spectrum is acquired by the analytical instrument with a different value of a parameter; converting each of the plurality of spectra into a graph as specified above; assessing the graphs to identify nodes representing peaks with corresponding peak positions; and adding, where peaks with corresponding peak positions are identified, at least one fourth edge connecting nodes from different graphs, wherein the nodes connected by the fourth edge represent peaks with corresponding peak positions.

The parameter may be normalised collision energy, polarity or ion activation.

The method may further comprise assigning different weights or labels to the at least one first edge and the fourth edge or edges. All fourth edges may have the same weight or label. The weight or label assigned to the respective fourth edges may depend on the value of the parameter, the respective peak intensity and the like.

The method may further comprise: at least a first mass spectrum and a second mass spectrum of the sample, wherein the first and second spectra respectively are acquired by the analytical instrument at a different MSn stage; converting the first and second spectra into a first and second graphs (the graphs comprising at least one node, each node representing, for a peak, peak position and peak intensity; and in case the respective spectrum comprises at least two peaks, at least one first edge connecting nodes representing neighbouring peaks in the spectrum);
assessing the first and second spectra and/or the first and second graphs to identify peaks, or nodes representing peaks, having corresponding peak positions in the first and second spectrum or the first and second graph, respectively; and adding, where peaks with corresponding peak positions are identified, at least one fifth edge connecting nodes from the first and second graphs, wherein the connected nodes represent, in a respective MS(n-1) stage, a precursor for the respective MSn stage.

The method may further comprise assigning different weights or labels to the at least one first edge and the fifth edge or edges. All first edges may have the same weight or label. All fifth edges may have the same weight or label. The fifth edges may have different weights or labels, depending e.g. on which precursor they are associated with, which MSn stage they are associated with, etc. The method may be further configured to assign different weights or labels to the first and/or second and/or third and/or fifth edge or edges, as appropriate.

The different embodiments of the method of the fourth aspect may be combined with each other as appropriate, even if such a combination is not explicitly stated above. The method of the fourth aspect may implement training analogous to the method of the third aspect and/or training suitable for producing a control unit as per the first aspect and/or training suitable for producing a control unit suitable for implementing the method of the third aspect.

In a fifth aspect, use of a control unit of the first aspect and/or the system of the second aspect and/or a method of the third aspect for one or both of the following is provided: identifying a sample composition (e.g. identifying at least one component of the sample) and/or identifying physical or chemical properties of at least one component of the sample.

The identification of the sample physical or chemical properties may be performed without first identifying the sample composition. The identification of the sample composition and/or sample component properties may be performed by a neural network trained by the method of the fourth aspect.

### BRIEF DESCRIPTION OF THE DRAWINGS

Fig 1 shows a schematic view of an analytical instrument and a processing unit;
Fig 2 shows a flow chart of a neural network training;
Fig 3 shows a flow chart of a trained neural network application;
Figs 4a and 4b show an example pattern present in mass spectrometry data;
Figs 5a to 5c show a schematic view of converting mass spectrometry data into input data for the neural network;
Fig 6 shows another example of converting mass spectrometry data into input data for the neural network;
Figs 7a and 7b show another example of converting mass spectrometry data into input data for the neural network;
Fig 8 shows another example of converting mass spectrometry data into input data for the neural network.

### DETAILED DESCRIPTION

In the following, a description of certain embodiments of the invention are given, with reference to the accompanying drawings. In the drawings, similar reference numerals may designate corresponding features. The description below is provided as an example only and it is not intended to limit the scope of the claims. Not all embodiments comprise all the features described below; new embodiments may be conceived by combining and/or omitting some of the features described below, as appropriate.

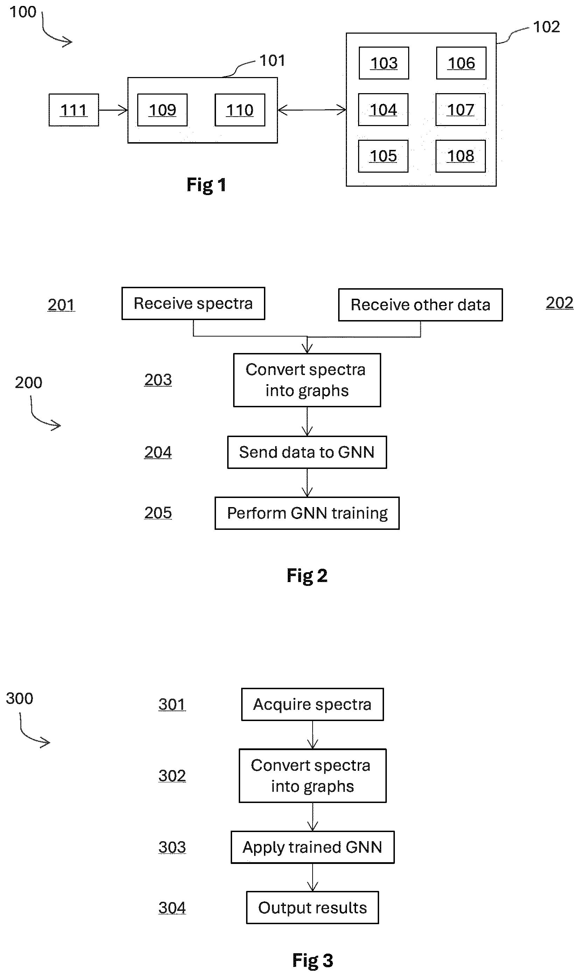

Fig 1 is a schematic block diagram of an example analytical system 100. The system 100 may comprise an analytical instrument 101 and a control unit 102. The control unit 102 may be a part of the instrument 101. The control unit 102 may be separate from the instrument 101 (external to the instrument 101) and may be connected to the instrument 101 by wired or wireless connection. The control unit 102 may be an external computer. There may be more than one control unit 102; for example, there may be a control unit controlling the analytical instrument and a control unit processing data (not shown).

The instrument 101 may be or may comprise a mass spectrometry, MS, instrument. The instrument 101 may employ one or more of the following methods: time-of-flight analysis, ion trapping, orbital ion trapping, multipole (e.g. quadrupole) mass filtering, Fourier transform mass spectrometry, or any other suitable method. The instrument 101 may be a part of a combination, e.g. a MS/MS or MSn instrument, a chromatography-MS instrument, LC-MS instrument, GC-MS instrument and the like, as appropriate. For the sake of brevity, the description below refers to the instrument 101 as a MS instrument and the performed method as mass spectrometry or MS analysis without further specification. It will be understood that any instruments, methods and combinations thereof outlined above may be used.

A typical MS instrument setup may comprise a sample introduction system 111 and the MS instrument 101. The sample introduction system 111 may be a part of the instrument 101, or it may be external to the instrument 101, as appropriate. The sample introduction system 111 transports a sample into the instrument 101. The instrument 101 may comprise first mass analyzer 109 and a second mass analyzer 110. In embodiments, more than two analytical components may be provided in the instrument 101. For example, appropriate component(s) may be provided for ion fragmentation (not shown), ion trapping (not shown) and other processes taking place prior to the ions being introduced into the first and/or second mass analyzer 109, 110. The instrument 101 may further comprise appropriate detector(s) (not shown).

In operation, the sample is fed into the instrument 101. The instrument 101 analyzes the ionized sample and outputs spectra, which generally show signal peaks for each mass-to-charge, *m*/*z,* ratio present in the sample. The spectra usually show noise as well as the signal peaks. The higher the intensity of a signal peak, the higher the incidence of the corresponding *m*/*z* ratio in the sample, and thus the higher the concentration of the respective sample component (element, ion, molecule or molecule fragment, as appropriate) corresponding to the given m/z ratio.

The spectra output by the instrument 101 are collected and processed by the control unit 102 to perform one or more of the following: identify the signal peaks, enhance the signal peaks, suppress the background noise, identify a specific m/z ratio associated with a particular signal peak, count the number of incidences of a specific m/z ratio, identify the presence of specific sample components (compounds, molecules, ions or their fragments, as appropriate), identify an approximate amount of specific sample components or their fragments.

The control unit 102 may comprise a CPU 103, a storage medium 104, a memory 105, a user interface 106, a network interface 107 and an instrument interface 108. The storage medium 104 may be a hard disk. The memory 105 may be a RAM. The storage medium 104 and/or the memory 105 may comprise instructions for controlling and/or operating the instrument 101. The CPU 103 may execute the instructions stored in the storage medium 104 and/or the memory 105. The network interface 107 may connect the control unit 102 to a wired or wireless network. The instrument interface 108 may connect the control unit 102 to the instrument 101, thus allowing the control unit 102 to control the instrument 101. The control unit 102 may comprise other components which are not shown. In some instances, the control unit 102 may omit some of the components described above. The control unit 102, among other instructions, may store data processing instructions, for example according to the methods described below.

In operation, the instrument 101 may be operated and the data may be processed according to an example method shown in Fig 2. For the sake of brevity, the example data processing method described below refers to components, which is intended to mean ions, compounds, molecules, or fragments thereof, as appropriate.

In processing of mass spectrometry data, neural networks may be used. The neural networks may be implemented by the control unit 102 and stored in the storage medium 104 and/or the memory 105. An example of a suitable neural network training method 200 is outlined in Fig 2. An example of a suitable application of a trained neural network (e.g. the neural network trained according to the method of Fig 2) in a data processing method 300 is shown in Fig 3.

Below, the method 200 is described as performed by the same control unit 102 as the method 300, but it will be understood that the method 200 may be performed by a different control unit (not shown). In such case, the data such as acquired spectra and the trained neural network output from the method 200 (step 205) may be transferred between the control unit 102 and the second control unit in any suitable way (e.g. wired or wireless transfer, a portable memory etc.).

In connection with Fig 2, two examples of the neural network training are described:
(i) a first example, which is training the neural network to predict one or more compound physical or chemical properties, e.g. toxicity, based on the mass spectra of the compound, and
(ii) a second example, which is training the neural network to perform and/or enhance compound identification based on not just position and intensity of peaks but also other data (metadata) present in the spectra.

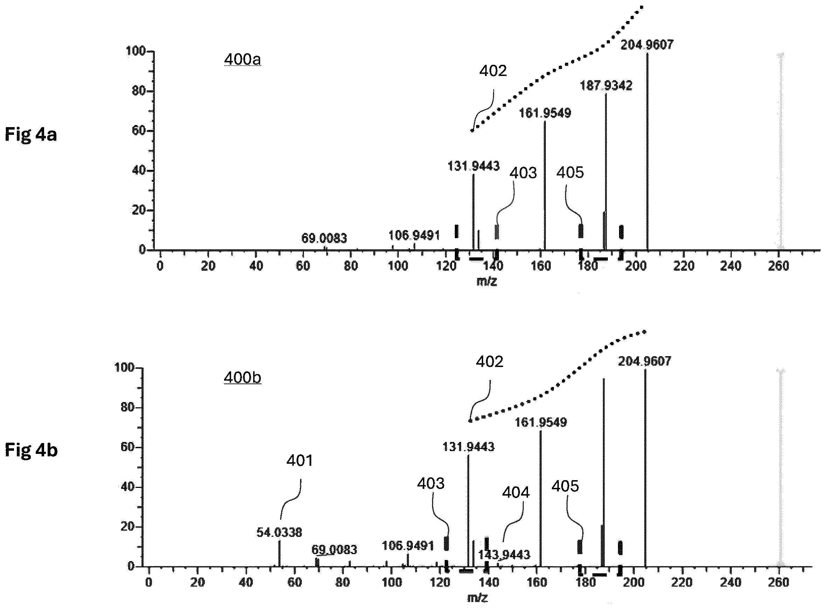

An example of metadata which could serve in the compound identification of the second example is shown in Figs 4a and 4b. Figs 4a and 4b show two spectra of the same compound. While the spectra 400a, 400b are not identical (e.g. spectrum 400b shows extra peaks 401, 404), the spectra 400a, 400b show similar patterns: an upward trend in peak intensity 402 and two peak pairs 403 and 405 (where, for each pair 403, 405, the lower-intensity peak in spectrum 400a and in spectrum 400b, respectively, shares the m/z ratio and a similar relative intensity to the neighbouring higher-intensity peak).

The method of Fig 2 starts with the control unit 102 receiving spectra in step 201. Spectra used for training may be obtained e.g. from an existing spectral library or database, from an acquisition by the instrument 101, or a combination thereof. In both examples described below (in particular in the second example), it may be desirable to train the neural network with the specific output of the instrument 101, and therefore the spectra received by the control unit 102 in step 201 may be spectra acquired by the instrument 101. In such case, the samples analyzed by the instrument 101 may be standard samples, i.e. samples containing known compounds in known concentrations.

In step 202, the control unit 102 receives other data for each spectrum received in step 201. In the first example, the other data may be properties corresponding to the compound the spectra of which has been received in step 201. In the second example, the other data may be library spectra corresponding to the standard samples analyzed by the instrument 101. The library spectra may be retrieved from an existing spectral library.

The steps 201 and 202 may be performed simultaneously and/or they may be merged into a single step in which the control unit 102 receives spectra and the corresponding other data.

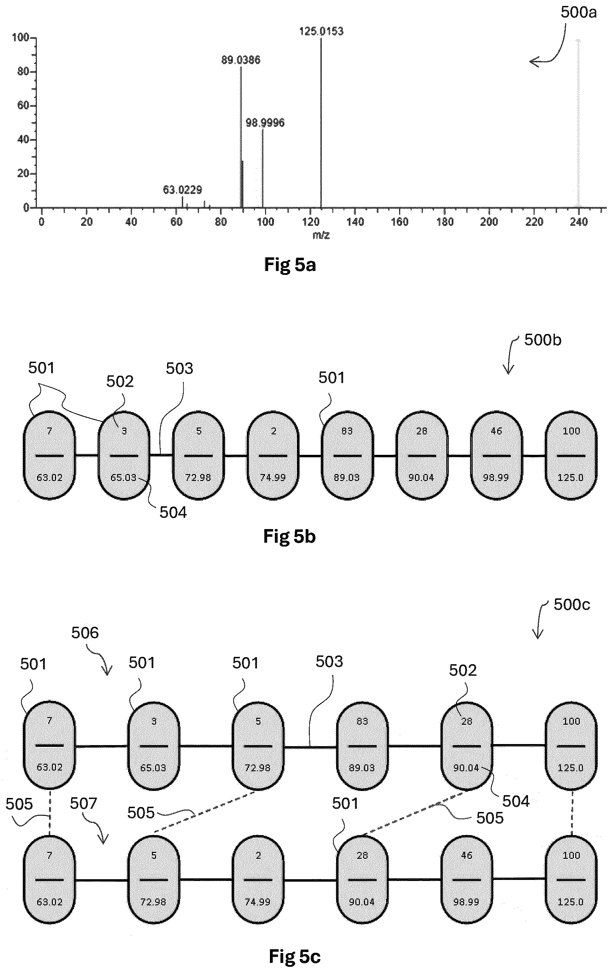

After receiving the spectra (step 201), the control unit 102 converts the spectra into graphs. This is schematically illustrated in Figs 5a to 5c, where Fig 5a shows an example mass spectrum 500a and Figs 5b, 5c show the spectrum of Fig 5a converted into graphs 500b, 500c respectively.

In a first example conversion (Fig 5b, graph 500b), each of the peaks of a compound spectrum 500a of Fig 5a is converted into a graph node 501. Each graph node has two values associated with the node: the peak intensity 502 and the peak position (m/z ratio) 504. The numbers 502, 504 (shown schematically as one above the other in Fig 5b) may be organized in a 2-tuple (·,·). The neighbouring nodes (representing neighbouring peaks) are connected by edges 503. In other words, each edge 503 represents the fact that the nodes 501 connected by the respective edge are neighbouring nodes.

In a second example conversion (Fig 5c, graph 500c), which is particularly suitable for the second example training, the acquired compound spectrum 500a is converted into a graph, analogously to the conversion described above in connection with the example of Fig 5b. The outcome is shown in Fig 5c as linear graph 506. The library spectrum (obtained in step 202) is also converted into a graph in the same way. The outcome is shown in Fig 5c as linear graph 507. Additional edges 505 are added to the graph 500c. The additional edges 505 connect nodes that represent the corresponding peaks in the acquired spectrum graph 506 and the library spectrum graph 507. In the example of Fig 5c, the peaks corresponding to the *m*/*z* ratios 63.02, 72.98 and 90.04 are connected by the additional edges 505 as corresponding peaks.

In step 204, the control unit 102 inputs the graph obtained in step 203 and (in the first example) the compound properties obtained in step 202 into a graph neural network, GNN. The GNN doesn't require any spectrum quality degradation (e.g. no *m*/*z* binning) as it is possible to input graphs of various sizes in the GNN.

Various GNN architectures may be used. Suitable architectures may be e.g. Graph Attention Networks or Message Passing Neural Networks. As outlined above, the aim of the GNN training is to train the GNN to either predict one or more compound properties, e.g. toxicity, based on the mass spectra of the compound, or to perform and/or enhance compound identification. The prediction or identification are both based on the graph representing the input spectrum.

An illustrative example of a GNN architecture suitable for the tasks outlined above could be a network with two graph attention layers with ReLU activation function, followed by a fully connected linear layer, as follows (the numbering of the following items is for ease of reference):
i. A graph attention layer with two input channels (as the input graph has two channels, the peak position and intensity, as discussed above); 8 output channels and 2 attention heads (then 2*8 = 16 will be the channel width of the hidden layers). For example, the implementation GATConv from the torch_geometric.nn module of the PyTorch Geometric library may be used.
ii. ReLU activation function.
iii. One more attention layer with 8 input channels, two heads and 16 output.
iv. ReLU activation function.
v. An aggregation function that collects the hidden node features into graph feature, for example taking the mean of the node features, for each channel separately.
vi. A fully connected layer with input size 16 and output 1.

Optionally, if the output values are known to have values only from the range [0,1], a sigmoid function may be applied as the last layer (i.e. after layer vi.).

In the above example, the number of hidden layers and the number of channels/heads in each layer may be adjusted as appropriate. Each layer may have a different number of channels. ReLU activation functions may be replaced with any suitable activation function, e.g. LeakyReLU.

As explained above, the aim of step 205 is to train the neural network to be able to receive a mass spectrum or a pair of mass spectra (in the form of a graph, as discussed above), and output (i) a prediction on one or more compound physical or chemical properties (e.g. toxicity) and/or (ii) an identification of the compound based on the received compound mass spectrum (and, in case (ii), also the received reference spectrum, e.g. from a spectra library). "Mass spectrum" in this context may mean a plurality of spectra acquired over multiple acquisitions and processed in an appropriate way and/or a plurality of reference spectra, identifying a plurality of candidate molecules and acquired from a spectra library.

These two aims (and the corresponding example methods) may be combined if/when appropriate. Both examples utilize pattern information from mass spectra that can be then further used to (i) predict physical or chemical properties from spectra without an intermediate step of recovering the compound identification, and/or (ii) enhance spectral library lookup by predicting if a spectrum pair (instrument acquired spectrum vs library spectrum) belongs to the same or similar compound.

The output of step 205 is a GNN trained to achieve aims (i) or (ii) above.

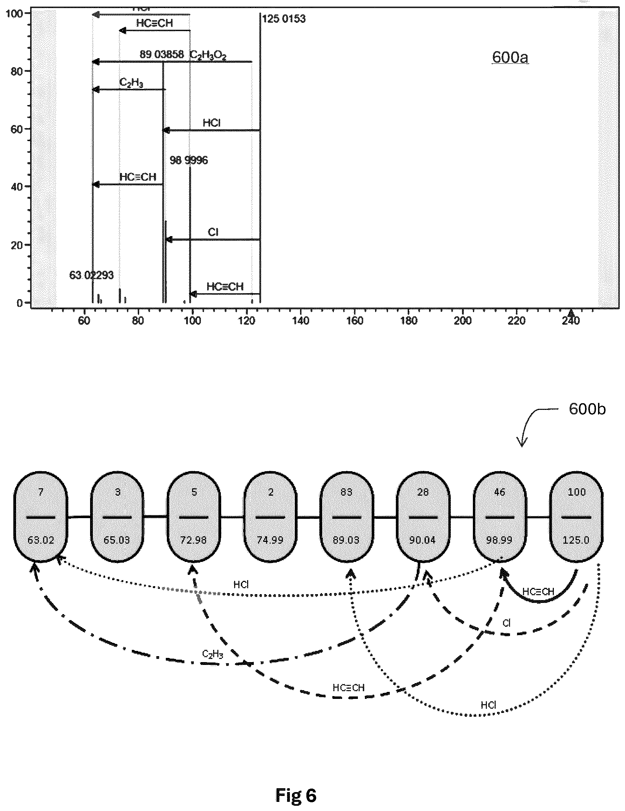

In embodiments, step 203 of the method 200 may be supplemented by the control unit 102 checking for and adding into the graph common neutral mass losses, as shown schematically in Fig 6. Before or after converting the spectrum into a graph, the control unit 102 may compare each peak (node) with other peaks (nodes). Based on the comparison, it may be assessed whether the mass (m/z) difference between the peaks (nodes) corresponds to a known common neutral mass loss (such as water or ammonia). An example outcome is shown in spectrum 600a. The spectrum 600a corresponds to the spectrum 500a of Fig 5, with the added annotations showing neutral mass loss. This type of information may be assessed and added by the control unit 102. The neutral mass loss information may be retrieved by the control unit 102 from a database or other suitable storage listing common neutral mass losses. The control unit 102 may utilize a list of common or predefined common neutral mass losses.

If this is the case and neutral mass loss is discovered, the neural mass loss may be translated by the control unit 102 into neutral mass loss edges, as shown in the graph 600b. Graph 600b adds to graph 500b of Fig 5b the edges representing the neutral mass loss. Each neutral mass loss edge is shown in a different line, to represent different labelling/weighing of the edges (where the labels or weights may represent e.g. the mass of the neutral mass loss). This provides additional pattern for the GNN to utilize in its training (step 205) and subsequent use (described below), and therefore may enhance the training and subsequent recognition results.

In embodiments, an acquisition of mass spectra of a sample by the instrument 101 may be repeated while varying a small number of parameters (e.g. a single parameter), and the output may be used to supplement the spectra-to-graph conversion of step 203. Alternatively or in addition, the parameters may be used as input into the ML model training and/or prediction, e.g. as additional inputs in the fully connected layer of step vi. of the example outlined above. The parameter may be e.g. normalised collision energy, polarity, ion activation, instrument data such as instrument and/or analyzer type etc.

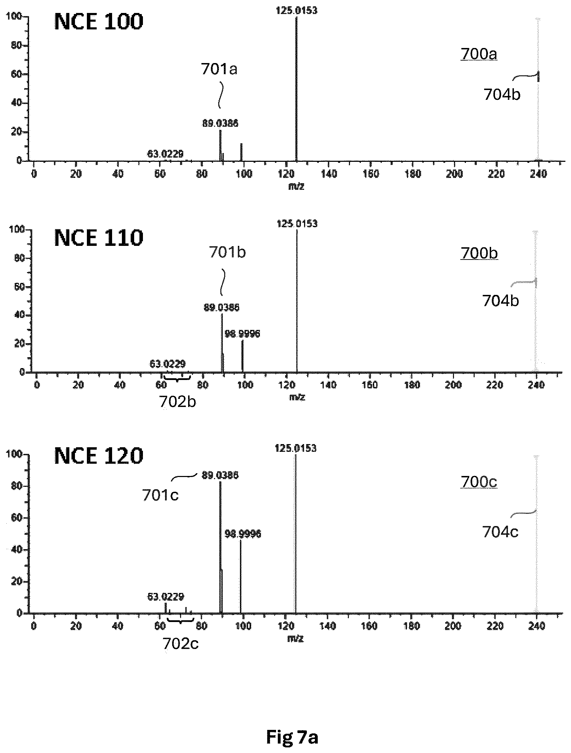

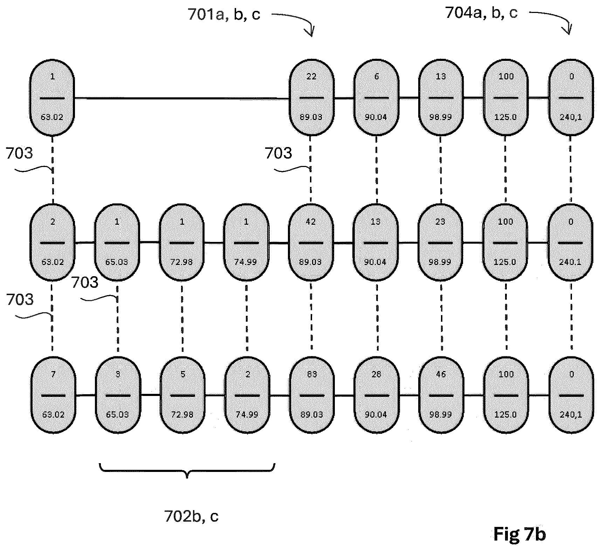

An example is shown in Figs 7a, 7b. In this example, specific (same) precursor and specific (same) ion activation method are used, and the three spectra 700a to 700c are shown correspond to varying normalised collision energy, NCE, levels. In spectra 700a-c, corresponding peaks show different intensities depending on the NCE. For example, the peak corresponding to *m*/*z* ratio of 89.0386 (labelled 701a-c in Fig 7a) shows a growing intensity with growing NCE; a group of peaks 702b, c are missing in the spectrum 700a, but appear in the spectra 700b, c where the NCE is higher; etc.

This information may be added into the graph obtained in step 203, as schematically shown in Fig 7b. Fig 7b shows each spectrum 700a-c converted into a linear graph, with edges 703 added between corresponding peaks in the graphs corresponding to each of the respective spectra 700a-c. The edges 703 may receive a different label/weight from the edges connecting the neighbouring peaks in the same spectrum. Precursor peaks 704a-c may be added in the spectrum graphs; if not present in the respective spectrum, they may be added with intensity 0. The NCE level may be added to the nodes, e.g. by forming a 3-tuple (·,·,·) of peak position (*m*/*z* ratio), peak intensity and NCE level.

While Fig 7 shows three different NCE levels, it will be understood that spectra for more than three NCE levels may be acquired and processed in the above-described way.

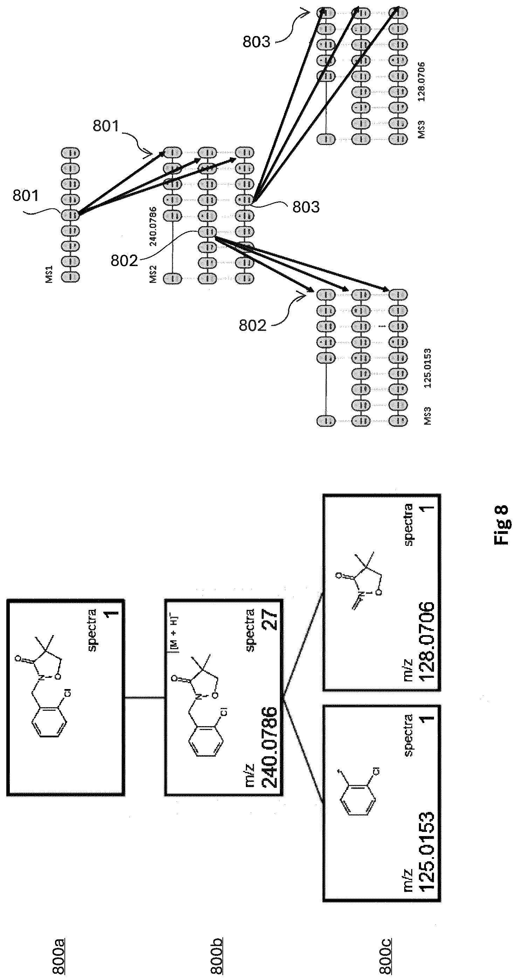

In generalisation of the above-described embodiments, a MSn-type experiment usually outputs a plurality of spectra for a single sample. The different spectra may correspond to different NCE levels, different MS stages (MS1, MS2 etc.), different adducts etc. In step 203, all these various spectra may be combined into one complex graph and subsequently used as an input for the GNN training (step 205 of method 200). For example, for MS1, the acquired spectrum may be converted into a single linear graph. For each subsequent MSn stage and each specific precursor mass, a subgraph may be constructed and then connected, via additional edges, to the corresponding nodes in the MS(n-1) and MSn graphs (e.g. from the precursor peak in the MS(n-1) graph to the corresponding peak in the MSn graph). This is shown schematically in Fig 8. The first level 800a corresponds to MS1. The second level 800b corresponds to MS2, with a single selected precursor 801 as the precursor and one linear graph per NCE level. The third level 800c corresponds to MS3, with two example precursors 802, 803 and one linear graph per NCE level.

It will be understood that Fig 8 is only provided for illustration purposes, and:
- multiple similar sets of MSn linear graphs may be constructed based on acquisition of mass spectra for different precursors from MS(n-1) stage, i.e. there may be a plurality of precursors and corresponding graphs;
- spectra may be acquired at more than three different NCE levels;
- different parameter than NCE level may be varied.

Once the neural network is trained, it may be used in data processing methods, an example of which is shown in Fig 3. As mentioned above, the method is described as this method being performed by the control unit 102, but it may be performed by a second control unit (not shown).

In step 301, the control unit 102 controls the analytical instrument 101 to acquire mass spectrum or spectra of a sample. The sample may be a sample the composition of which and/or the properties of which are not known (or known precisely). The sample may be output from another analytical instrument, e.g. chromatography instrument, a mass filter or mass spectrometer (e.g. in a MSn-type experiment). The spectra acquisition may be based on considerations such as the extent to which the GNN was previously trained on same or similar type of spectra. For example, with a GNN trained on spectra and corresponding graphs as shown in Fig 8 and described above, analogous MSn spectra for different precursors and with varying levels of NCE or other parameter(s) may be acquired by the instrument 101 to provide the GNN with more input information. Similarly, for GNN trained on spectra and corresponding graphs as shown in Figs 5 to 7, corresponding spectra and graphs may be acquired by the instrument 101.

After spectra acquisition, the control unit 102 in step 302 converts the acquired spectra into graphs, in a manner analogous to that outlined above in connection with step 203 of method 200 and its various embodiments described above in connection with Figs 5 to 8. The graph conversion of this step may be based on considerations involving training data, as described in connection with step 301.

Once spectra are converted into graphs, the control unit 102, in step 303, applies the trained GNN to the spectra, and then outputs the results of the GNN in step 304. The results may depend on the specific training (e.g. according to first example, second example or both examples outlined above in connection with step 205), the specific task (e.g. the desired outcome in case the GNN is capable of both tasks according to the first and second examples), etc. In an example, the GNN may output predicted properties of the compound(s) present in the sample, e.g. toxicity, without the intermediate step of determining the exact sample composition. In another example, the GNN may output an identification of compound(s) present in the sample based at least partly on the metadata present in the spectra and reflected in the graphs used as input for the GNN.

The input data for the GNN (on which the GNN is applied in step 303) may depend on the extent the GNN was previously trained on same type of data, as described above in connection with steps 301 and 302.

## Claims

1. A control unit for processing data acquired by an analytical instrument, the control unit configured to:
receive at least one mass spectrum of a sample, wherein the mass spectrum is acquired by an analytical instrument and comprises at least one peak having a peak position, represented by a *m*/*z* ratio, and a peak intensity;
convert the at least one mass spectrum into a graph, the graph comprising:
at least one node, each node representing, for a peak, peak position and peak intensity; and
in case the spectrum of a sample comprises at least two peaks, at least one first edge connecting nodes representing neighbouring peaks in the spectrum;
apply a trained graph neural network on the graph; and
output the result of the application of the trained graph neural network.

2. The control unit of claim 1, wherein the result comprises at least one of the following:
at least one candidate component of the sample;
at least one physical or chemical property of a component of the sample.

3. The control unit of claim 1 or claim 2, wherein the control unit is further configured to:
assess whether, for each pair of peaks, the difference in peak positions corresponds to any one of a list of neutral mass losses, and
in case a pair of nodes is assessed to represent peaks for which the difference in positions corresponds to a neutral mass loss, add a second edge into the graph connecting the pairs of nodes.

4. The control unit of claim 3, wherein the control unit is further configured to assign different weights or labels to the at least one first edge and the second edge or edges.

5. The control unit of any one of preceding claims, wherein the control unit is further configured to:
receive a plurality of mass spectra of the sample, wherein each spectrum is acquired by the analytical instrument with a different value of a parameter;
convert each of the plurality of spectra into a graph comprising:
at least one node, each node representing, for a peak, peak position and peak intensity; and
in case the spectrum of a sample comprises at least two peaks, at least one first edge connecting nodes representing neighbouring peaks in the spectrum;
assess the graphs to identify nodes representing peaks with corresponding peak positions; and
add, where peaks with corresponding peak positions are identified, at least one third edge connecting nodes from different graphs, wherein the nodes connected by the third edge represent peaks with corresponding peak positions.

6. The control unit of claim 5, wherein the parameter is normalised collision energy, polarity or ion activation.

7. The control unit of claim 5 or claim 6, wherein the control unit is further configured to assign a different weight or a different label to the at least one first edge and to the at least one third edge.

8. The control unit of any one of preceding claims, wherein the control unit is further configured to:
receive at least a first mass spectrum and a second mass spectrum of the sample, wherein the first and second spectra respectively are acquired by the analytical instrument at a different MSn stage;
convert the first and second spectra into a first and second graphs comprising:
at least one node, each node representing, for a peak, peak position and peak intensity; and
in case the respective spectrum comprises at least two peaks, at least one first edge connecting nodes representing neighbouring peaks in the spectrum;
assess the first and second spectra and/or the first and second graphs to identify peaks, or nodes representing peaks, having corresponding peak positions in the first and second spectrum or the first and second graph, respectively; and
add, where peaks with corresponding peak positions are identified, at least one fourth edge connecting nodes from the first and second graphs, wherein the connected nodes represent, in a respective MS(n-1) stage, a precursor for the respective MSn stage.

9. The control unit of any one of preceding claims, wherein the control unit is further configured to control an analytical instrument to acquire mass spectra.

10. A system comprising an analytical instrument and a control unit of any one of claims 1 to 9.

11. A method of processing data acquired by an analytical instrument, the method comprising:
receiving, by a control unit, at least one mass spectrum of a sample, wherein the mass spectrum is acquired by an analytical instrument and comprises peaks, each peak having a peak position, represented by a *m*/*z* ratio, and a peak intensity;
converting, by the control unit, the at least one mass spectrum into a graph, the graph comprising:
at least one node, each node representing, for a peak, peak position and peak intensity; and
in case the spectrum of a sample comprises at least two peaks, at least one first edge connecting nodes representing neighbouring peaks in the spectrum;
applying, by the control unit, a trained graph neural network on the graph; and
outputting, by the control unit, the result of the application of the trained graph neural network.

12. A method of training a graph neural network to process data acquired by an analytical instrument, the method comprising:
receiving, by a control unit, at least one mass spectrum, wherein the mass spectrum comprises peaks, each peak having a peak position, represented by a *m*/*z* ratio, and a peak intensity;
receiving, by a control unit, other data for the at least one mass spectrum;
converting, by the control unit, the at least one mass spectrum into a graph, the graph comprising:
at least one node, each node representing, for a peak, peak position and peak intensity; and
in case the spectrum of a sample comprises at least two peaks, at least one first edge connecting nodes representing neighbouring peaks in the spectrum;
applying, by the control unit, the graph neural network on the graph and the other data.

13. The method of training a graph neural network of claim 12, wherein the other data is at least one physical or chemical property of a compound represented by the mass spectrum.

14. The method of training a graph neural network of claim 12 or claim 13, wherein the at least one mass spectrum is a mass spectrum of a sample acquired by an analytical instrument, and the other data is at least one library spectrum corresponding to a sample of the same composition.

15. The method of training a graph neural network of claim 14, further comprising:
converting, by the control unit, the at least one library spectrum into a graph comprising:
at least one node, each node representing, for a peak, peak position and peak intensity; and
in case the spectrum of a sample comprises at least two peaks, at least one second edge connecting nodes representing neighbouring peaks in the spectrum;
assessing, by the control unit, the at least one mass spectrum of a sample and the at least one library spectrum and/or the respective graphs to identify peaks and/or nodes represent peaks with corresponding peak positions; and
adding, by the control unit, at least one third edge connecting nodes from the respective graphs, wherein the nodes connected by the third edge represent peaks with corresponding peak positions.
